# EUROPEAN PATENT SPECIFICATION

(11) **EP 4 486 438 B1**
(45) Date of publication and mention of the grant of the patent: **09.09.2026**
(21) Application number: 23706370.6
(22) Date of filing: 27.02.2023
(51) Int. Cl.: A61N 5/06, H05B 45/20, C09K 11/00

(54) **THREE CHANNEL CHIP-ON-BOARD WITH TUNABLE MELANOPIC ACTIVITY AT CONSTANT COLOR POINT**
DREIKANALIGES CHIP-ON-BOARD MIT ABSTIMMBARER MELANOPISCHER AKTIVITÄT BEI KONSTANTEM FARBPUNKT
PUCE INTÉGRÉE À TROIS CANAUX AVEC ACTIVITÉ MÉLANOPIQUE RÉGLABLE À POINT DE COULEUR CONSTANT

(30) Priority: 03.03.2022 EP 22160015; 29.03.2022 EP 22164935
(43) Date of publication of application: 08.01.2025
(73) Proprietor: Signify Holding B.V., 5656 AE Eindhoven (NL)
(72) Inventor: PEETERS, Martinus, Petrus, Joseph, 5656 AE Eindhoven (NL); WEGH, René, Theodorus, 5656 AE Eindhoven (NL)
(74) Representative: Verweij, Petronella Daniëlle
(86) International application number: PCT/EP2023/054786
(87) International publication number: WO 2023/165917

(56) References cited:
- WO-A1-2021/204934
- US-B2- 10 750 591

## Description

### FIELD OF THE INVENTION

The invention relates to a light generating system. The invention also relates to a lighting device comprising such light generating system.

### BACKGROUND OF THE INVENTION

The impact of light on the circadian rhythm is known in the art. For instance, WO2016145064 describes an apparatus for effecting a circadian outcome for an individual, the apparatus including an article of eyewear that disposed relative to one or both eyes of an individual, the article of eyewear having one or more filter elements configured to controllably attenuate spectral components of light incident on the eyewear; the spectral components are in circadian-active wavelength ranges; and the one or more filter elements are controlled based on at least information associated with a circadian outcome of the individual.

WO2021/204934A1 discloses a light generating system generating system comprises a first string with first and second light generating devices, and a second string with third light generating devices. The first light generating devices comprise (i) a first light source generating first light source light having a first dominant wavelength selected from the range of 470-500 nm, and (ii) a first luminescent material converting part of the first light source light into first luminescent material light. The light from the first light generating has a first color point and a first correlated color temperature. The second and third light generating device comprise (i) a blue light source, and (ii) a luminescent material configured to convert at least part of the light from the solid-state light source into luminescent material light. The light from the second light generating device is white light having a second color point and a second correlated color temperature. The light from the third light generating device is white light having a third color point and a third correlated color temperature. The color point and hence the correlated color temperature of the first and second light generating device are comparable. The first and second correlated color temperature are at least 700 K larger than the third correlated color temperature. The first string provides relatively cool white light, whereas the second string provides relatively warm white light.

US10750591B discloses a method for generating tunable white light with controllable circadian energy performance. The method uses a plurality of LED strings to generate light with color points that fall within blue, yellow/green, red, and cyan color ranges, with each LED string being driven with a separately controllable drive current in order to tune the generated light output. Different light emitting modes can be selected that utilize different combinations of the plurality of LED strings in order to tune the generated white light.

### SUMMARY OF THE INVENTION

There is a desire for light generating systems allowing to provide light with a tunable Melanopic Daylight Efficacy Ratio (MDER) value. Further, there appears to be a desire to provide light with a substantially fixed correlated color temperature (CCT) but having a variable MDER. Yet further, there appears to be a desire to provide light with a substantially (fixed) CCT but having a variable MDER, while the fixed CCT may be selectable from a relatively large temperature range. Further, for certain applications a high CRI may be desirable. Prior art systems, however, do not provide such functionality / functionalities.

Hence, it is an aspect of the invention to provide an alternative light generating system, which preferably further at least partly obviates one or more of above-described drawbacks. The present invention may have as object to overcome or ameliorate at least one of the disadvantages of the prior art, or to provide a useful alternative. The invention is defined by the claims.

The invention provides a light generating system, configured to generate system light, wherein the light generating system comprises a first SSL light source string, a second SSL light source string and a third SSL light source string, wherein: (A) the first SSL light source string is configured to generate first light having a first color point and a first correlated color temperature, and which comprises at least one first SSL light source for generating violet and/or blue light and a first luminescent material; (B) the second SSL light source string is configured to generate second light having a second color point and a second correlated color temperature, and which comprises at least one second SSL light source for generating violet and/or blue light and a second luminescent material; (C) the third SSL light source string is configured to generate third light having a third color point and a third correlated color temperature, and which comprises at least one third SSL light source for generating cyan light and a third luminescent material; (D) the first correlated color temperature is lower than the second correlated color temperature; and (E) the first correlated color temperature is lower than the third correlated color temperature. In yet more specific embodiments, the invention provides a light generating system, configured to generate system light, wherein the light generating system comprises a first SSL light source string, a second SSL light source string and a third SSL light source string, wherein: (A) the first SSL light source string is configured to generate light having a first color point, a first correlated color temperature and a first MDER value, and which comprises at least one first SSL light source for generating violet and/or blue light and a first luminescent material; (B) the second SSL light source string is configured to generate light having a second color point, a second correlated color temperature and a second MDER value, and which comprises at least one second SSL light source for generating violet and/or blue light and a second luminescent material; (C) the third SSL light source string is configured to generate light having a third color point, a third correlated color temperature and a third MDER value, and which comprises at least one third SSL light source for generating cyan light and a third luminescent material; (D) the first correlated color temperature is lower than the second correlated color temperature; and (E) the first correlated color temperature is lower than the third correlated color temperature.

With such system, it is possible to provide light with a high color rendering index with variable correlated color temperature and variable MDER. Further, with such system it is possible to vary the MDER value while keeping the color point essentially constant. Yet, with such system it is possible to vary the MDER value while keeping the CCT essentially constant, while the fixed CCT may be selectable out a relatively large temperature range.

As indicated above, the light generating system is especially configured to generate system light. The system light may comprise one or more of the first light, the second light, and the third light. Other sources of light are herein not excluded but are herein not further discussed (in detail). In embodiments, the system light may consist of one or more of the first light, the second light, and the third light.

The term "system light" may especially refer to light escaping from the system. In embodiments, the system may comprise a light exit, like an end window or an (other) optical element, or an opening, from which the system light may escape to the external of the system. The system may comprise a housing, comprising such light exit. The housing may at least partly enclose one or more light sources and one or more (other) optical elements.

Especially, the light generating system may comprise a first SSL light source string, a second SSL light source string and a third SSL light source string.

SSL light strings, especially LED strings, are known in the art, and for instance described in US9474111B2 or US8035315B2. As indicated by US8035315B2, the term "LED string," may refer to a grouping of one or more LEDs connected in series. The "head end" of a LED string is the end or portion of the LED string which receives the driving voltage/current and the "tail end" of the LED string is the opposite end or portion of the LED string.

The strings are individually controllable. To this end the system may comprise a control system or may be functionally coupled to a control system. See further also below in relation to embodiments of the control system and/or about operational modes. Therefore, in embodiments, the light generating system may further comprises a control system configured to individually control the first SSL light source string, the second SSL light source string, and the third SSL light source string. Especially, in this way the system light may be controlled. Hence, in embodiments the control system may be configured to control the system light by controlling the first light, the second light, and the third light. In specific embodiments, the control system may be configured to control one or more of the correlated color temperature, the color point, and the Melanopic Daylight Efficacy Ratio of the system light.

In embodiments, the control system may be configured to control in an operational mode the system light such that the color point of the system light may be maintained constant, and the Melanopic Daylight Efficacy Ratio is varied. Hence, when varying the MDER, the color point at different MDER values may essentially be the same. In specific embodiments, colors, or color points of a first type of light and a second type of light may be essentially the same when the respective color points of the first type of light and the second type of light differ with at maximum 0.03 for u' and/or with at maximum 0.03 for v', even more especially at maximum 0.02 for u' and/or with at maximum 0.02 for v'. In yet more specific embodiments, the respective color points of first type of light and the second type of light may differ with at maximum 0.01 for u' and/or with at maximum 0.01 for v'. Here, u' and v' are color coordinate of the light in the CIE 1976 UCS (uniform chromaticity scale) diagram. See further below for further embodiments in relation to MDER.

In embodiments, the control system may be configured to control in an operational mode the system light such that the CCT of the system light may be maintained constant and the Melanopic Daylight Efficacy Ratio is varied. For instance, the variation in CCT may be less than 5% of a predetermined CCT at which the MDER may be controlled and/or within about +/- 300 K of the predetermined CCT at which the MDER may be controlled.

Yet, in embodiments the control system may be configured to control in an operational mode the system light such that the CRI of the system light stays above a predefined threshold, such as at least 80, in specific embodiments even at least 85, while allowing control of e.g. one or more of the CCT and the MDER.

Yet, in embodiments the control system may be configured to control in an operational mode the system light such that the R9 of the system light stays above a predefined threshold, such as at least 0, in specific embodiments even at least 20, such as even at least about 40, while allowing control of e.g. one or more of the CCT and the MDER.

The solid state light sources (SSL light sources) of the respective strings provide, during operation of the (respective) light source, light which may at least partly be converted by the respective luminescent material(s). This may lead to the respective light, which light comprises luminescent material light and optionally unconverted light of the respective solid state light source of the respective string.

The first SSL light source string may comprise a first light source and a first luminescent material, which may especially be configured to convert at least part of the light of the first light source into first luminescent material light. Hence, in embodiments the first SSL light source string may be configured to generate first light having a first color point and a first correlated color temperature CCT1, and which comprises at least one first SSL light source for generating violet and/or blue light and a first luminescent material. The first luminescent material may especially be configured to convert at least part of the light generated by the at least one first SSL light source.

The second SSL light source string may comprise a second light source and a second luminescent material, which may especially be configured to convert at least part of the light of the second light source into second luminescent material light. Hence, in embodiments the second SSL light source string may be configured to generate second light having a second color point and a second correlated color temperature CCT2, and which comprises at least one second SSL light source for generating violet and/or blue light and a second luminescent material. The second luminescent material may especially be configured to convert at least part of the light generated by the at least one second SSL light source.

The third SSL light source string may comprise a third light source and a third luminescent material, which may especially be configured to convert at least part of the light of the third light source into third luminescent material light. Hence, in embodiments the third SSL light source string is configured to generate third light having a third color point and a third correlated color temperature CCT3, and which comprises at least one third SSL light source for generating cyan light and a third luminescent material. The third luminescent material may especially be configured to convert at least part of the light generated by the at least one third SSL light source.

Hence, whereas the first SSL light source string and the second SSL light source string may comprise one or more light sources configured to generate light source light having wavelengths in the blue and/or UV, the third SSL light source string may comprise one or more light sources configured to generate light source light having wavelengths in the cyan.

According to the invention : (i) the first correlated color temperature is lower than the second correlated color temperature, and (ii) the first correlated color temperature is lower than the third correlated color temperature. The first SSL light source string is configured to generate warm-white light, and the second SSL light source string and the third SSL light source string are configured to generate cold-white light.

In specific embodiments, CCT3>CCT2>CCT1.

In embodiments, one or more of the following, especially all, may apply: (i) CCT3> 3000 K, (ii) CCT2> 3000 K, and (iii) CCT1≤2700 K.

In embodiments, one or more of the following, especially all, may apply: (i) CCT3-CCT1≥1000 K, (ii) CCT2-CCT1≥1000 K, and (iii) CCT3-CCT2≥1000 K.

Yet, in more specific embodiments one or more of the following, especially all, may apply: (i) CCT3-CCT1≥2000 K, (ii) CCT2-CCT1≥2000 K, and (iii) CCT3-CCT2≥2000 K.

Yet, in more specific embodiments one or more of the following, especially all, may apply: (i) CCT3> 6000 K, (ii) CCT2> 5000 K, and (iii) CCT1≤2500 K.

In specific embodiments, the first correlated color temperature may differ from the second correlated color temperature and the third correlated color temperature with at least 500 K, more especially at least 1000 K, such as in embodiments, at least 1500 K, such as at least about 2000 K.

In embodiments, the system light may thus be white light. Especially, the first light may be white light, the second light may be white light, and the third light may be white light. In this way, essentially any combination or sole provision of the first light, the second light, and the third light, may lead to white system light. Other embodiments, however, are herein not excluded.

The term "white light", and similar terms, herein, is known to the person skilled in the art. It may especially relate to light having a correlated color temperature (CCT) between about 1800 K and 20000 K, such as between 2000 and 20000 K, especially 2700-20000 K, for general lighting especially in the range of about 2000-7000 K, such as in the range of 2200-6500 K, like especially 2700 K and 6500 K. In embodiments, e.g. for backlighting purposes, or for other purposes, the correlated color temperature (CCT) may especially be in the range of about 7000 K and 20000 K. Yet further, in embodiments the correlated color temperature (CCT) is especially within about 15 SDCM (standard deviation of color matching) from the BBL (black body locus), especially within about 10 SDCM from the BBL, even more especially within about 5 SDCM from the BBL.

The system light is substantially white light with a correlated color temperature in the range between 2200 - 6500 K, preferably in the range of 2700 - 6500 K, more preferably in the range of 2200 - 5000 K.

According to the invention, the first color point, the second color point, and the third color point are on a straight line in a color diagram.

Especially, in embodiments all three color points of the first light, the second light, and the third light, may essentially be on a straight line in the color diagram, such as especially the 1931 CIE x,y color diagram, or the CIE 1976 UCS u',v' diagram.

In embodiments, the color points may be determined on the basis of the 10° color matching functions, such as described in International Standard ISO/CIE 11664-1, Colorimetry - Part 1: CIE standard colorimetric observers, 2019-06, which is herein incorporated by reference.

According to an example not part of the invention, the The three color points do not need to be exactly on a straight line in e.g. the color diagram 1931 CIE, but may also be configured at some distance. In general, however, this distance from the straight line may not be more than 10 SDCM, such as not more than 7 SDCM, such as in specific embodiments not more than 5 SDCM. In specific embodiments, this distance from the straight line may not be more than 3 SDCM. Hence, in specific embodiments, selecting a straight line through two color points, and having the straight line intercept the 10 SDCM range around the other color point, especially the 7 SDCM range, more especially the 4 SDCM range, may provide of a set of color points in compliance with the present invention.

The first, second and third color point do not overlap within 10 standard deviation of color matching (SDCM), preferably do not overlap within 7 standard deviation of color matching (SDCM). As indicated above, two of the three color points are from light having CCTs that may differ at least 1000 K, more especially at least 1500 K.

Hence, in specific embodiments the first correlated color temperature may differ from the second correlated color temperature and the third correlated color temperature with at least 1000 K. Further, all three color points, determined on the basis of 10° color matching functions may be positioned within 10 standard deviation of color matching (SDCM) of the substantially straight line in the color diagram, and at least two of the three color points within 10 standard deviation of color matching (SDCM) do not overlap. Hence, in embodiments the respective 10 SDCM ranges of at least two of the three color points do not overlap.

The term "light source" may in principle relate to any light source known in the art. It may be a conventional (tungsten) light bulb, a low pressure mercury lamp, a high pressure mercury lamp, a fluorescent lamp, a LED (light emissive diode). In a specific embodiment, the light source comprises a solid state LED light source (such as a LED or laser diode (or "diode laser")). The term "light source" may also relate to a plurality of light sources, such as 2-2000 (solid state) LED light sources. Hence, the term LED may also refer to a plurality of LEDs. Further, the term "light source" may in embodiments also refer to a so-called chips-on-board (COB) light source. The terms "COB" or "CoB" may especially refer to LED chips in the form of a semiconductor chip that is neither encased nor connected but directly mounted onto a substrate, such as a PCB. Hence, a plurality of light emitting semiconductor light source may be configured on the same substrate. In embodiments, a COB is a multi LED chip configured together as a single lighting module.

The light source may have a light escape surface. Referring to conventional light sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

Likewise, a light generating device may comprise a light escape surface, such as an end window. Further, likewise a light generating system may comprise a light escape surface, such as an end window.

The term "light source" may refer to a semiconductor light-emitting device, such as a light emitting diode (LEDs), a resonant cavity light emitting diode (RCLED), a vertical cavity laser diode (VCSELs), an edge emitting laser, etc... The term "light source" may also refer to an organic light-emitting diode (OLED), such as a passive-matrix (PMOLED) or an active-matrix (AMOLED). In a specific embodiment, the light source comprises a solid-state light source (such as a LED or laser diode). In an embodiment, the light source comprises a LED (light emitting diode). The terms "light source" or "solid state light source" may also refer to a superluminescent diode (SLED). Especially, the term "solid state light source" or "SSL light source" may refer to LEDs.

The term LED may also refer to a plurality of LEDs.

The term "solid state light source" or "SSL light source" may also relate to a plurality of (essentially identical (or different)) light sources, such as 2-2000 light sources. In embodiments, the light source may further comprise one or more micro-optical elements (array of micro lenses) downstream of the light source, or downstream of a plurality of light sources (i.e. e.g. shared by multiple light sources). An SSL light source may comprise one or more of an LED, a superluminescent diode, a laser diode, a laser or a laser bank, amongst others. The term "SSL" especially refers to solid state light or solid state lighting.

In embodiments, the light source may comprise a LED with on-chip optics. In embodiments, the light source comprises a pixelated single LEDs (with or without optics) (offering in embodiments on-chip beam steering).

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs.

In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as a LED with a luminescent material layer or dome comprising luminescent material. Such LEDs may be indicated as phosphor converted LEDs or PC LEDs (phosphor converted LEDs). In other embodiments, the luminescent material may be configured at some distance ("remote") from the light source, such as a LED with a luminescent material layer not in physical contact with a die of the LED. Hence, in specific embodiments the light source may be a light source that during operation emits at least light at wavelength selected from the range of 380-470 nm. However, other wavelengths may also be possible. This light may partially be used by the luminescent material.

The light source may especially be configured to generate light source light having an optical axis (O), (a beam shape,) and a spectral power distribution. The light source light may in embodiments comprise one or more bands, having band widths as known for lasers.

The term "light source" may (thus) refer to a light generating element as such, like e.g. a solid state light source, or e.g. to a package of the light generating element, such as a solid state light source, and one or more of a luminescent material comprising element and (other) optics, like a lens, a collimator. A light converter element ("converter element" or "converter") may comprise a luminescent material comprising element. For instance, a solid state light source as such, like a blue LED, is a light source. A combination of a solid state light source (as light generating element) and a light converter element, such as a blue LED and a light converter element, optically coupled to the solid state light source, may also be a light source (but may also be indicated as light generating device). Hence, a white LED is a light source (but may e.g. also be indicated as (white) light generating device).

The term "light source" herein may also refer to a light source comprising a solid state light source, such as an LED or a laser diode or a superluminescent diode.

The "term light source" may (thus) in embodiments also refer to a light source that is (also) based on conversion of light, such as a light source in combination with a luminescent converter material. Hence, the term "light source" may also refer to a combination of a LED with a luminescent material configured to convert at least part of the LED radiation, or to a combination of a (diode) laser with a luminescent material configured to convert at least part of the (diode) laser radiation.

In embodiments, the term "light source" may also refer to a combination of a light source, like a LED, and an optical filter, which may change the spectral power distribution of the light generated by the light source. Especially, the "term light generating device" may be used to address a light source and further (optical components), like an optical filter and/or a beam shaping element, etc.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The term "solid state light source", or "solid state material light source", and similar terms, may especially refer to semiconductor light sources, such as a light emitting diode (LED), a diode laser, or a superluminescent diode.

In embodiments, the first SSL light source may be configured to provide first SSL light having a first dominant wavelength λ1d selected from the range of 440-470 nm, such as selected from the range of 450-460 nm, and/or having a first centroid wavelength λ1c selected from the range of 435-470 nm, such as selected from the range of 445-460 nm.

Alternatively, or additionally, in embodiments the second SSL light source is configured to second SSL light having a second dominant wavelength λ2d selected from the range of 425-470 nm, such as selected from the range of 435-460 nm, and/or having a second centroid wavelength λ2c selected from the range of 425-465 nm, such as selected from the range of 435-455 nm.

Alternatively, or additionally, in embodiments the third SSL light source is configured to third SSL light having a third dominant wavelength λ3d selected from the range of 470-520 and/or having a third centroid wavelength λ3c selected from the range of 465-520 nm, such as selected from the range of 470-520, like selected from the range of 470-515 nm.

Especially, in embodiments (i) λ3d>λ2d and λ3d>λ1d, and/or (ii) λ3c>λ2c and λ3c>λ1c (may apply). More especially, in embodiments (i) λ3d-λ2d≥10 nm and λ3d-λ1d≥10 nm, and/or (ii) λ3c-λ2c≥10 nm and λ3c-λ1c≥10 nm (may apply). Yet, in specific embodiments (i) λ3d-λ2d≥30 nm and/or λ3c-λ2c≥30 nm (may apply).

The term "centroid wavelength", also indicated as λc, is known in the art, and refers to the wavelength value where half of the light energy is at shorter and half the energy is at longer wavelengths; the value is stated in nanometers (nm). It is the wavelength that divides the integral of a spectral power distribution into two equal parts as expressed by the formula λc = Σ λ*I(λ) / (Σ I(λ)), where the summation is over the wavelength range of interest, and I(λ) is the spectral energy density (i.e. the integration of the product of the wavelength and the intensity over the emission band normalized to the integrated intensity). The centroid wavelength may e.g. be determined at operation conditions.

The term "dominant wavelength" may refer to the wavelength of the monochromatic stimulus that, when additively mixed in suitable proportions with the specified achromatic stimulus, matches the color stimulus considered.

Herein, the invention may be explained in relation to embodiments wherein the system comprises a chip-on-board device (see also above). Hence, in embodiments the light generating system may comprise a chip-on-board device, wherein the chip-on-board-device comprises the first SSL light source string, the second SSL light source string, the third SSL light source string, the first luminescent material, the second luminescent material, and the third luminescent material (and optionally the fourth luminescent material, etc.).

Especially, the first luminescent material may be configured downstream of substantially only the first SSL light source(s). Especially, the second luminescent material may be configured downstream of substantially only the second SSL light source(s). Especially, the third luminescent material may be configured downstream of substantially only the third SSL light source(s).

However, as indicated also elsewhere, the first luminescent material, the second luminescent material, and the third luminescent material may be (essentially) identical. Hence, a luminescent material may be configured downstream of (i) one or more of the first SSL light sources, especially downstream of all, (ii) one or more of the second SSL light sources, especially downstream of all, and (iii) one or more of the third SSL light sources, especially downstream of all.

In further embodiments, see also below, the system may comprise a fourth luminescent material configured downstream of one or more of the first SSL light sources, especially downstream of all (and not downstream of the second and/or third SSL light sources). Therefore, in embodiments the chip-on-board-device may (also) comprise the fourth luminescent material.

In specific embodiments the chip-on-board-device may be obtainable by: (i) providing a printed circuit board comprising the first SSL light source string, the second SSL light source string, and the third SSL light source string; (ii) providing the fourth luminescent material to the first SSL light source and not to the second SSL light source or the third SSL light source; and (iii) providing the first luminescent material, the second luminescent material, and the third luminescent material to the first SSL light source (and fourth luminescent material), the second SSL light source, and the third SSL light source, respectively.

Therefore, in an aspect the invention also provides a method comprising: (i) providing a printed circuit board comprising a first SSL light source string (especially as defined herein), a second SSL light source string (especially as defined herein), and a third SSL light source string (especially as defined herein); (ii) providing a fourth luminescent material (especially as defined herein) to the first SSL light source (especially comprised by the first string) and not to the second SSL light source (especially comprised by the second string) or the third SSL light source (especially comprised by the third string); and (iii) providing the first luminescent material (especially as defined herein), the second luminescent material (especially as defined herein), and the third luminescent material (especially as defined herein) to the first SSL light source (and fourth luminescent material), the second SSL light source, and the third SSL light source, respectively. Hence, with only two deposition steps, the CoB may already be provided, instead of three deposition steps. The latter may especially apply when the first luminescent material, the second luminescent material, and the third luminescent material may be identical.

The first luminescent material, the second luminescent material and the third luminescent material may be substantially identical or may be different. As amongst others indicated above, in embodiments, the first luminescent material, the second luminescent material, and the third luminescent material may be substantially identical.

The light generating system may be chip-on-board (CoB) type of system. The luminescent material at least partly converts the light from the SSL light source.

In an embodiment, the first color point, the second color point, and the third color point are on a substantially straight (single) line in a color diagram. The color points may be expressed as u' and v' color coordinates of the light in the CIE 1976 UCS (uniform chromaticity scale) diagram. Alternatively, the color points may be expressed as coordinates in the CIE1931 color space chromaticity diagram.

The term "luminescent material" especially refers to a material that can convert first radiation, especially one or more of violet radiation, cyan radiation, and blue radiation, into second radiation. In general, the first radiation and second radiation have different spectral power distributions. Hence, instead of the term "luminescent material", also the terms "luminescent converter" or "converter" may be applied. In general, the second radiation has a spectral power distribution at larger wavelengths than the first radiation, which is the case in the so-called down-conversion. In specific embodiments, however the second radiation has a spectral power distribution with intensity at smaller wavelengths than the first radiation, which is the case in the so-called up-conversion. In embodiments, the "luminescent material" may especially refer to a material that can convert radiation into e.g. visible and/or infrared light. For instance, in embodiments the luminescent material may be able to convert one or more of blue radiation, violet radiation and cyan radiation into visible light.

Hence, upon excitation with radiation, the luminescent material emits radiation. In general, the luminescent material will be a down converter, i.e. radiation of a smaller wavelength is converted into radiation with a larger wavelength (λₑₓ<λₑₘ), though in specific embodiments the luminescent material may comprise up-converter luminescent material, i.e. radiation of a larger wavelength is converted into radiation with a smaller wavelength (λₑₓ>λₑₘ).

In embodiments, the term "luminescence" may refer to phosphorescence. In embodiments, the term "luminescence" may also refer to fluorescence. Instead of the term "luminescence", also the term "emission" may be applied. Hence, the terms "first radiation" and "second radiation" may refer to excitation radiation and emission (radiation), respectively. Likewise, the term "luminescent material" may in embodiments refer to phosphorescence and/or fluorescence.

The term "luminescent material" may also refer to a plurality of different luminescent materials. Examples of possible luminescent materials are indicated below. Hence, the term "luminescent material" may in specific embodiments also refer to a luminescent material composition.

In embodiments, luminescent materials are selected from garnets and nitrides, especially doped with trivalent cerium or divalent europium, respectively. The term "nitride" may also refer to oxynitride or nitridosilicate, etc. Alternatively, or additionally, the luminescent material(s) may be selected from silicates, especially doped with divalent europium.

In specific embodiments the luminescent material comprises a luminescent material of the type A₃B₅O₁₂:Ce, wherein A in embodiments comprises one or more of Y, La, Gd, Tb and Lu, especially (at least) one or more of Y, Gd, Tb and Lu, and wherein B in embodiments comprises one or more of Al, Ga, In and Sc. Especially, A may comprise one or more of Y, Gd and Lu, such as especially one or more of Y and Lu. Especially, B may comprise one or more of Al and Ga, more especially at least Al, such as essentially entirely Al. Hence, especially suitable luminescent materials are cerium comprising garnet materials. Embodiments of garnets especially include A₃B₅O₁₂ garnets, wherein A comprises at least yttrium or lutetium and wherein B comprises at least aluminum. Such garnets may be doped with cerium (Ce), with praseodymium (Pr) or a combination of cerium and praseodymium; especially however with Ce. Especially, B comprises aluminum (Al), however, B may also partly comprise gallium (Ga) and/or scandium (Sc) and/or indium (In), especially up to about 20% of Al, more especially up to about 10% of Al (i.e. the B ions essentially consist of 90 or more mole % of Al and 10 or less mole % of one or more of Ga, Sc and In); B may especially comprise up to about 10% gallium. In another variant, B and O may at least partly be replaced by Si and N. The element A may especially be selected from the group consisting of yttrium (Y), gadolinium (Gd), terbium (Tb) and lutetium (Lu). Further, Gd and/or Tb are especially only present up to an amount of about 20% of A. In a specific embodiment, the garnet luminescent material comprises (Y₁₋ₓLuₓ)₃B₅O₁₂:Ce, wherein x is equal to or larger than 0 and equal to or smaller than 1. The term ":Ce", indicates that part of the metal ions (i.e. in the garnets: part of the "A" ions) in the luminescent material is replaced by Ce. For instance, in the case of (Y₁₋ₓLuₓ)₃Al₅O₁₂:Ce, part of Y and/or Lu is replaced by Ce. This is known to the person skilled in the art. Ce will replace A in general for not more than 10%; in general, the Ce concentration will be in the range of 0.1 to 4%, especially 0.1 to 2% (relative to A). Assuming 1% Ce and 10% Y, the full correct formula could be (Y_{0.1}Lu_{0.89}Ce_{0.01})₃Al₅O₁₂. Ce in garnets is substantially or only in the trivalent state, as is known to the person skilled in the art.

In embodiments, the luminescent material (thus) comprises A₃B₅O₁₂ wherein in specific embodiments at maximum 10% of B-O may be replaced by Si-N.

In specific embodiments the luminescent material comprises (Yₓ₁₋ₓ₂₋ₓ₃A'ₓ₂Ceₓ₃)₃(Al_{y1-y2}B'_{y2})₅O₁₂, wherein x1+x2+x3=1, wherein x3>0, wherein 0<x2+x3≤0.2, wherein y1+y2=1, wherein 0≤y2≤0.2, wherein A' comprises one or more elements selected from the group consisting of lanthanides, and wherein B' comprises one or more elements selected from the group consisting of Ga, In and Sc. In embodiments, x3 is selected from the range of 0.001-0.1. In the present invention, especially x1>0, such as >0.2, like at least 0.8. Garnets with Y may provide suitable spectral power distributions.

In specific embodiments at maximum 10% of B-O may be replaced by Si-N. Here, B in B-O refers to one or more of Al, Ga, In and Sc (and O refers to oxygen); in specific embodiments B-O may refer to Al-O. As indicated above, in specific embodiments x3 may be selected from the range of 0.001-0.04. Especially, such luminescent materials may have a suitable spectral distribution (see however below), have a relatively high efficiency, have a relatively high thermal stability, and allow a high CRI (in combination with the light source light). Hence, in specific embodiments A may be selected from the group consisting of Lu and Gd. Alternatively or additionally, B may comprise Ga. Hence, in embodiments the luminescent material comprises (Yₓ₁₋ₓ₂₋ₓ₃(Lu,Gd)ₓ₂Ceₓ₃)₃(Al_{y1-y2}Ga_{y2})₅O₁₂, wherein Lu and/or Gd may be available. Even more especially, x3 is selected from the range of 0.001-0.1, wherein 0<x2+x3≤0.1, and wherein 0≤y2≤0.1. Further, in specific embodiments, at maximum 1% of B-O may be replaced by Si-N. Here, the percentage refers to moles (as known in the art); see e.g. also EP3149108. In yet further specific embodiments, the luminescent material comprises (Yₓ₁₋ₓ₃Ceₓ₃)₃Al₅O₁₂, wherein x1+x3=1, and wherein 0<x3≤0.2, such as 0.001-0.1.

In specific embodiments, one or more luminescent materials may be selected from the type of cerium comprising garnets. In even further specific embodiments, one of the solid state light sources may include a single type of luminescent materials, such as (Yₓ₁₋ₓ₂₋ₓ₃A'ₓ₂Ceₓ₃)₃(Al_{y1-y2}B'_{y2})₅O₁₂. Hence, in specific embodiments the light source may comprise luminescent material, wherein at least 85 weight %, even more especially at least about 90 wt.%, such as yet even more especially at least about 95 weight % of the luminescent material comprises (Yₓ₁₋ₓ₂₋ₓ₃A'ₓ₂Ceₓ₃)₃(Al_{y1-y2}B'_{y2})₅O₁₂. Here, wherein A' comprises one or more elements selected from the group consisting of lanthanides, and wherein B' comprises one or more elements selected from the group consisting of Ga, In, and Sc, wherein x1+x2+x3=1, wherein x3>0, wherein 0<x2+x3≤0.2, wherein y1+y2=1, wherein 0≤y2≤0.2. Especially, x3 is selected from the range of 0.001-0.1. Note that in embodiments x2=0. Alternatively or additionally, in embodiments y2=0.

In specific embodiments, A may especially comprise at least Y, and B may especially comprise at least Al.

Alternatively, or additionally, wherein the luminescent material may comprises a luminescent material of the type A₃Si₆N₁₁:Ce³⁺, wherein A comprises one or more of Y, La, Gd, Tb and Lu, such as in embodiments one or more of La and Y.

In embodiments, the luminescent material may alternatively or additionally comprise one or more of MS:Eu²⁺ and/or M₂Si₅N₈:Eu²⁺ and/or MAlSiN₃:Eu²⁺ and/or Ca₂AlSi₃O₂N₅:Eu²⁺, etc., wherein M comprises one or more of Ba, Sr and Ca, especially in embodiments at least Sr. Hence, in embodiments, the luminescent may comprise one or more materials selected from the group consisting of (Ba,Sr,Ca)S:Eu, (Ba,Sr,Ca)AlSiN₃:Eu and (Ba,Sr,Ca)₂Si₅N₈:Eu. In these compounds, europium (Eu) is substantially or only divalent, and replaces one or more of the indicated divalent cations. In general, Eu will not be present in amounts larger than 10% of the cation; its presence will especially be in the range of about 0.5 to 10%, more especially in the range of about 0.5 to 5% relative to the cation(s) it replaces. The term ":Eu", indicates that part of the metal ions is replaced by Eu (in these examples by Eu²⁺). For instance, assuming 2% Eu in CaAlSiN₃:Eu, the correct formula could be Ca_{0.98}Eu_{0.02})AlSiN₃. Divalent europium will in general replace divalent cations, such as the above divalent alkaline earth cations, especially Ca, Sr or Ba. The material (Ba,Sr,Ca)S:Eu can also be indicated as MS:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Further, the material (Ba,Sr,Ca)₂Si₅N₈:Eu can also be indicated as M₂Si₅N₈:Eu, wherein M is one or more elements selected from the group consisting of barium (Ba), strontium (Sr) and calcium (Ca); especially, M comprises in this compound Sr and/or Ba. In a further specific embodiment, M consists of Sr and/or Ba (not taking into account the presence of Eu), especially 50 to 100%, more especially 50 to 90% Ba and 50 to 0%, especially 50 to 10% Sr, such as Ba_{1.5}Sr_{0.5}Si₅N₈:Eu (i.e. 75 % Ba; 25% Sr). Here, Eu is introduced and replaces at least part of M, i.e. one or more of Ba, Sr, and Ca). Likewise, the material (Ba,Sr,Ca)AlSiN₃:Eu can also be indicated as MAlSiN₃:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Eu in the above indicated luminescent materials is substantially or only in the divalent state, as is known to the person skilled in the art.

In embodiments, a red luminescent material may comprise one or more materials selected from the group consisting of (Ba,Sr,Ca)S:Eu, (Ba,Sr,Ca)AlSiN₃:Eu and (Ba,Sr,Ca)₂Si₅N₈:Eu. In these compounds, europium (Eu) is substantially or only divalent, and replaces one or more of the indicated divalent cations. In general, Eu will not be present in amounts larger than 10% of the cation; its presence will especially be in the range of about 0.5 to 10%, more especially in the range of about 0.5 to 5% relative to the cation(s) it replaces. The term ":Eu", indicates that part of the metal ions is replaced by Eu (in these examples by Eu²⁺). For instance, assuming 2% Eu in CaAlSiN₃:Eu, the correct formula could be (Ca_{0.98}Eu_{0.02})AlSiN₃. Divalent europium will in general replace divalent cations, such as the above divalent alkaline earth cations, especially Ca, Sr or Ba.

The material (Ba,Sr,Ca)S:Eu can also be indicated as MS:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Further, the material (Ba,Sr,Ca)₂Si₅N₈:Eu can also be indicated as M₂Si₅N₈:Eu, wherein M is one or more elements selected from the group consisting of barium (Ba), strontium (Sr) and calcium (Ca); especially, M comprises in this compound Sr and/or Ba. In a further specific embodiment, M consists of Sr and/or Ba (not taking into account the presence of Eu), especially 50 to 100%, more especially 50 to 90% Ba and 50 to 0%, especially 50 to 10% Sr, such as Ba_{1.5}Sr_{0.5}Si₅N₈:Eu (i.e. 75 % Ba; 25% Sr). Here, Eu is introduced and replaces at least part of M, i.e. one or more of Ba, Sr, and Ca). Likewise, the material (Ba,Sr,Ca)AlSiN₃:Eu can also be indicated as MAlSiN₃:Eu, wherein M is one or more elements selected from the group consisting of barium (Ba), strontium (Sr) and calcium (Ca); especially, M comprises in this compound calcium or strontium, or calcium and strontium, more especially calcium. Here, Eu is introduced and replaces at least part of M (i.e. one or more of Ba, Sr, and Ca). Eu in the above indicated luminescent materials is substantially or only in the divalent state, as is known to the person skilled in the art.

Blue luminescent materials may comprise YSO (Y₂SiO₅:Ce³⁺), or similar compounds, or BAM (BaMgAl₁₀O₁₇:Eu²⁺), or similar compounds.

The term "luminescent material" herein especially relates to inorganic luminescent materials. Instead of the term "luminescent material" also the term "phosphor". These terms are known to the person skilled in the art.

Alternatively or additionally, also other luminescent materials may be applied. For instance, quantum dots and/or organic dyes may be applied and may optionally be embedded in transmissive matrices like e.g. polymers, like PMMA, or polysiloxanes, etc. etc. Quantum dots are small crystals of semiconducting material generally having a width or diameter of only a few nanometers. When excited by incident light, a quantum dot emits light of a color determined by the size and material of the crystal. Light of a particular color can therefore be produced by adapting the size of the dots. Most known quantum dots with emission in the visible range are based on cadmium selenide (CdSe) with a shell such as cadmium sulfide (CdS) and zinc sulfide (ZnS). Cadmium free quantum dots such as indium phosphide (InP), and copper indium sulfide (CuInS₂) and/or silver indium sulfide (AgInS₂) can also be used. Quantum dots show very narrow emission band and thus they show saturated colors. Furthermore, the emission color can easily be tuned by adapting the size of the quantum dots. Any type of quantum dot known in the art may be used in the present invention. However, it may be preferred for reasons of environmental safety and concern to use cadmium-free quantum dots or at least quantum dots having a very low cadmium content. Instead of quantum dots or in addition to quantum dots, also other quantum confinement structures may be used. The term "quantum confinement structures" should, in the context of the present application, be understood as e.g. quantum wells, quantum dots, quantum rods, tripods, tetrapods, or nano-wires, etcetera. Organic phosphors can be used as well. Examples of suitable organic phosphor materials are organic luminescent materials based on perylene derivatives, for example compounds sold under the name Lumogen^{®} by BASF. Examples of suitable compounds include, but are not limited to, Lumogen^{®} Red F305, Lumogen^{®} Orange F240, Lumogen^{®} Yellow F083, and Lumogen^{®} F 170.

Different luminescent materials may have different spectral power distributions of the respective luminescent material light. Alternatively or additionally, such different luminescent materials may especially have different color points (or dominant wavelengths).

As indicated above, other luminescent materials may also be possible. Hence, in specific embodiments the luminescent material is selected from the group of divalent europium containing nitrides, divalent europium containing oxynitrides, divalent europium containing silicates, cerium comprising garnets, and quantum structures. Quantum structures may e.g. comprise quantum dots or quantum rods (or other quantum type particles) (see above). Quantum structures may also comprise quantum wells. Quantum structures may also comprise photonic crystals.

In specific embodiments, the first luminescent material may comprise a luminescent material of the type A₃B₅O₁₂:Ce, wherein A comprises one or more of Y, La, Gd, Tb and Lu, and wherein B comprises one or more of Al, Ga, In and Sc. Alternatively or additionally, the second luminescent material may also comprise a luminescent material of the type A₃B₅O₁₂:Ce, wherein A comprises one or more of Y, La, Gd, Tb and Lu, and wherein B comprises one or more of Al, Ga, In and Sc. However, the first luminescent material and the (optional) second luminescent material may especially be selected such that at irradiation with the light source light the respective luminescent material light (of the first luminescent material and the second luminescent material) have different spectral power distributions.

The luminescent material may be chosen such that an emission band of a full width half maximum (of the luminescent material light) of at least 40 nm, such as at least 50 nm is obtained. For instance, the luminescent material may be chosen such that an emission band of a full width half maximum of at least 60 nm, is obtained. This may e.g. be the case with trivalent cerium comprising garnet luminescent materials (as described herein). Hence, especially the luminescent material may comprise a broad band emitter. The luminescent material may also comprise a plurality of broad band emitters. Especially, when two or more luminescent materials are applied to convert at least part of the first device light and/or at least part of the second device light, at least two of the two or more luminescent materials may be configured to provide respective luminescent material light each having an emission band with full width half maximum (of the luminescent material light) of at least 40 nm, such as at least 50 nm.

In embodiments, the first luminescent material, second luminescent material, and third luminescent material may be substantially identical, more especially identical. In such embodiments, a single luminescent material may be used which represents the first luminescent material, the second luminescent material, and the third luminescent material (but not the fourth luminescent material). Examples of such luminescent materials include Ce-doped garnets (such as e.g. (Lu_{1-x-y-a-b}YₓGd_{y})₃(Al_{1-z-u}Ga_{z}Siᵤ)₅O₁₂₋ᵤNᵤ:CeₐPr_{b}), Eu-doped silicates (such as e.g. (Sr₁₋ₓBaₓ)₂SiO₄:Eu), Eu-doped nitrides (such as e.g. SCASN: (Ca_{1-x-y}Sr_{y})AlSiN₃:Euₓ²⁺) or a combination of two or more thereof. Especially, the first luminescent material may comprise a combination of (i) a green and/or yellow emitting luminescent material, especially at least green emitting luminescent material, and (ii) a red emitting luminescent material.

In relation to (Lu_{1-x-y-a-b}YₓGd_{y})₃Al₅O₁₂N:CeₐPr_{b}), it is noted that 0<a≤0.2, especially 0.005≤a≤0.1; 0≤b≤0.2, especially 0≤b≤0.05, such as b<0.00001; 0≤x<1; 0≤y<1; 0≤x+y+b<1; 0≤x+y+a+b≤1. In embodiments, part of Al-O may be replaced by Si-N.

In relation to M₂SiO₄:Eu, such as (Sr₁₋ₓBaₓ)₂SiO₄:Eu), it is noted that 0≤x≤1 (may apply). Further, relative to Eu in these compounds, it is noted that europium is in the divalent state and may be available in the range of 0.5-10 at% relative to M (in the example Sr₁₋ₓBaₓ).

In relation to MAlSiN₃:Eu, such as Ca_{1-x-y}Sr_{y})AlSiN₃:Euₓ²⁺, it is noted that relative to Eu in these compounds, europium is in the divalent state and may be available in the range of 0.5-10 at% relative to M (in the example Ca_{1-x-y}Sr_{y}). Further, in relation to as Ca_{1-x-y}Sr_{y})AlSiN₃:Euₓ²⁺, it is noted that 0<x+y≤1 and 0≤y<1 (may apply).

Hence, in embodiments the light generating system may further comprise a fourth luminescent material different from the first, second and third luminescent material, wherein the fourth luminescent material is configured downstream of the at least one first SSL light source and may not be configured downstream of the at least one second SSL light source and the at least one third SSL light source.

Hence, in embodiments the first SSL light source string may comprise a first light source and the first luminescent material and the fourth luminescent material, which may especially be configured to convert at least part of the light of the first light source into first luminescent material light and fourth luminescent material light. Hence, in embodiments the first SSL light source string may be configured to generate first light having a first color point and a first correlated color temperature CCT1, and which comprises at least one first SSL light source for generating violet and/or blue light and the first luminescent material and the fourth luminescent material. The first light may thus comprise fourth luminescent material light, first luminescent material light, and optionally light of the first light source, especially all three contributions.

In specific embodiments, the first luminescent material, the second luminescent material, and third luminescent material may be configured downstream of all of the at least one first SSL light source, the at least one second SSL light source, and the at least one third SSL light source. Further, especially in embodiments there is no other luminescent material configured downstream of the at least one second SSL source and the at least one third SSL light source. As indicated above, the first luminescent material, the second luminescent material, and third luminescent material may in embodiments be essentially identical.

Especially, the first SSL light source string may further comprise the fourth luminescent material different from the first, second and third luminescent material. In specific embodiments, the fourth luminescent material may be an orange and/or red light emitting luminescent material. In embodiments, the fourth luminescent material may comprise Ce-doped garnets (see also above), Eu-doped silicates (see also above), Eu-doped nitrides see also above) or a combination of two or more thereof.

In embodiments, the first luminescent material, the second luminescent material, and the third luminescent material are essentially identical, and comprise a first combination of at least two luminescent materials, at least one configured to generate red light and at least one configured to generate green and/or yellow light. Hence, this combination of luminescent materials may be configured downstream of all of the at least one first SSL light source, the at least one second SSL light source, and the at least one third SSL light source. Yet further, the fourth luminescent material comprise a second combination at least two luminescent materials, at least one configured to generate red light and at least one configured to generate green and/or yellow light. Especially, the fourth luminescent material may be configured downstream of the at least one first SSL light source (and not configured downstream of the at least one second SSL light source and/or not configured downstream of the at least one third SSL light source). Hence, the fourth luminescent material may be configured downstream of one or more of the first SSL light sources, especially downstream of all (and not downstream of the second and/or third SSL light sources). Especially, the spectral power distribution of the fourth luminescent material light may differ from the spectral power distribution of the first luminescent material light, and from the spectral power distribution of the second luminescent material light, and from the spectral power distribution of the third luminescent material light. Hence, in embodiments the first combination of luminescent materials differs from the second combination of luminescent materials. The difference may be in the relative amounts and/or in the chemical composition of the luminescent materials, especially at least the latter. The term "combination of luminescent materials" may especially refer to a mixture of luminescent materials.

In specific embodiments, the second SSL light source string may further comprise a fifth luminescent material.

Hence, in embodiments the second SSL light source string may comprise a second light source and the second luminescent material and the fifth luminescent material, which may especially be configured to convert at least part of the light of the second light source into second luminescent material light and fifth luminescent material light. Hence, in embodiments the second SSL light source string may be configured to generate second light having a second color point and a second correlated color temperature CCT2, and which comprises at least one second SSL light source for generating violet and/or blue light and the second luminescent material and the fifth luminescent material. The second light may thus comprise fifth luminescent material light, second luminescent material light, and optionally light of the second light source, especially all three contributions. In embodiments, the fifth luminescent material may comprise Ce-doped garnets (see also above), Eu-doped silicates (see also above), Eu-doped nitrides see also above) or a combination of two or more thereof.

Yet further, the fifth luminescent material comprise a second combination at least two luminescent materials, at least one configured to generate red light and at least one configured to generate green and/or yellow light. Especially, the fifth luminescent material may be configured downstream of the at least one second SSL light source (and not configured downstream of the at least one first SSL light source and/or not configured downstream of the at least one third SSL light source). Especially, the spectral power distribution of the fifth luminescent material light may differ from the spectral power distribution of the first luminescent material light, and from the spectral power distribution of the second luminescent material light, and from the spectral power distribution of the third luminescent material light, and from the spectral power distribution of the fourth luminescent material light.

As indicated above, the present invention allows - amongst others - controlling the MDER value, such as controlling this value while keeping the color point of the system light essentially constant and/or while keeping the CCT of the system light essentially constant.

The Melanopic Daylight Efficacy Ratio, abbreviated to "melanopic DER" or "MDER", may be used to indicate the melanopsin active radiation in a flux of the light. In words, the melanopic DER is the ratio of the melanopic efficacy of luminous radiation (for a source), to the melanopic efficacy of luminous radiation for daylight (D65). In formula: $melanopic DER = {γ}_{mel , v}^{D 65} = \left(\frac{{Φ}_{mel}}{{Φ}_{v}}\right) / \left(\frac{{Φ}_{mel}^{D 65}}{{Φ}_{v}^{D 65}}\right)$ in which *Φₘₑₗ* represents the melanopic radiant flux and *Φᵥ* the luminous flux. In equation 2, the superscript indicates the illuminant, being either the source (no superscript) or daylight (D65). When the source is daylight D65, the melanopic DER equals 1. Further, we have ${Φ}_{mel} = \int SPD \left(λ\right) {s}_{mel} \left(λ\right) dλ$ ${Φ}_{v} = {K}_{m} \int SPD \left(λ\right) V \left(λ\right) dλ$ with *SPD* (*λ*) the spectral power distribution of the source, *sₘₑₗ*(*λ*) representing the action spectrum of ipRGCs due to their photopigment melanopsin ( =m(λ) ), and *V*(*λ*) the photopic luminous efficiency function, *Kₘ* is the maximum spectral luminous efficacy of radiation for photopic vision, *K*ₘ = 683 lm·W⁻¹. The *sₘₑₗ*(*λ*) and *V*(*λ*) functions are shown in the Table on page 34 and onwards. The denominator in equation 2 is a constant with the value of 0.001362, hence equation 2 may be simplified to: $melanopic DER = \left(\frac{{Φ}_{mel}}{{Φ}_{v}}\right) / 0.001362 = 754.03 \left(\frac{{Φ}_{mel}}{{Φ}_{v}}\right)$

In embodiments, the first light may have a first Melanopic Daylight Efficacy Ratio (MDER), the second light has a second Melanopic Daylight Efficacy Ratio (MDER), and the third light has a third Melanopic Daylight Efficacy Ratio (MDER), wherein in specific embodiments the second MDER value may be lower than the third MDER value.

In embodiments, the first MDER may be selected from a range of 0.065-1.02, such as 0.07-1.015. Alternatively or additionally, the second MDER may be selected from a range of 0.25-1.7, such as 0.35-1.6. Yet, alternatively or additionally, the third MDER may be selected from a range of 1.20-2.4, such as 1.30-2.25.

In embodiments, the third MDER value may be larger than both the first MDER value and the second MDER value. Especially, in embodiments at least two of the first MDER value, the second MDER value, and the third MDER value may mutually differ at least 0.1.

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions from a remote control. In embodiments, the control system may be controlled via an App on a device, such as a portable device, like a Smartphone or I-phone, a tablet, etc. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, WIFI, LiFi, ZigBee, BLE or WiMAX, or another wireless technology.

The system, or apparatus, or device may execute an action in a "mode" or "operation mode" or "operational mode" or "mode of operation" or "control mode". Likewise, in a method an action or stage, or step may be executed in a "mode" or operation mode" or "operational mode" or "mode of operation" or "control mode". The term "mode" may also be indicated as "controlling mode". This does not exclude that the system, or apparatus, or device may also be adapted for providing another controlling mode, or a plurality of other controlling modes. Likewise, this may not exclude that before executing the mode and/or after executing the mode one or more other modes may be executed. The terms "operational mode", or "an operational mode", and similar terms, may refer (in embodiments) to one or more operational modes.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

As indicated above, the luminescent material may partly convert the light source light. This may be the case in a transmissive mode or in a reflective mode. The non-converted light source light may in embodiment propagate together with the first luminescent material light.

The terms "visible", "visible light" or "visible emission" and similar terms refer to light having one or more wavelengths in the range of about 380-780 nm. Herein, UV may especially refer to a wavelength selected from the range of 200-380 nm. The terms "light" and "radiation" are herein interchangeably used, unless clear from the context that the term "light" only refers to visible light. The terms "light" and "radiation" may thus refer to UV radiation, visible light, and IR radiation. In specific embodiments, especially for lighting applications, the terms "light" and "radiation" refer to (at least) visible light. The terms "violet light" or "violet emission" especially relates to light having a wavelength in the range of about 380-440 nm. The terms "blue light" or "blue emission" especially relates to light having a wavelength in the range of about 440-495 nm (including some violet and cyan hues). The terms "green light" or "green emission" especially relate to light having a wavelength in the range of about 495-570 nm. The terms "yellow light" or "yellow emission" especially relate to light having a wavelength in the range of about 570-590 nm. The terms "orange light" or "orange emission" especially relate to light having a wavelength in the range of about 590-620 nm. The terms "red light" or "red emission" especially relate to light having a wavelength in the range of about 620-780 nm. The term "cyan" may refer to one or more wavelengths selected from the range of about 470-520 nm. The term "amber" may refer to one or more wavelengths selected from the range of about 585-605 nm, such as about 590-600 nm.

The term "white light" herein, is known to the person skilled in the art. It especially relates to light having a correlated color temperature (CCT) between about 1800 K and 20000 K, such as between 2000 and 20000 K, especially 2700-20000 K, for general lighting especially in the range of about 2700 K and 6500 K. In embodiments, for backlighting purposes the correlated color temperature (CCT) may especially be in the range of about 7000 K and 20000 K. Yet further, in embodiments the correlated color temperature (CCT) is especially within about 15 SDCM (standard deviation of color matching) from the BBL (black body locus), especially within about 10 SDCM from the BBL, even more especially within about 5 SDCM from the BBL.

Especially, in embodiments the control system may be configured to control one or more of the color rendering index, the correlated color temperature and the MDER value of the system light.

The light generating system may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting. The light generating system (or luminaire) may be part of or may be applied in e.g. optical communication systems or disinfection systems.

In yet a further aspect, the invention also provides a lamp or a luminaire comprising the light generating system as defined herein. The luminaire may further comprise a housing, optical elements, louvres, etc. etc. The lamp or luminaire may further comprise a housing enclosing the light generating system. The lamp or luminaire may comprise a light window in the housing or a housing opening, through which the system light may escape from the housing. In yet a further aspect, the invention also provides a projection device comprising the light generating system as defined herein. Especially, a projection device or "projector" or "image projector" may be an optical device that projects an image (or moving images) onto a surface, such as e.g. a projection screen. The projection device may include one or more light generating systems such as described herein. Hence, in an aspect the invention also provides a lighting device selected from the group of a lamp, a luminaire and a projector device comprising the light generating system as defined herein. The lighting device may comprise a housing or a carrier, configured to house or support, one or more elements of the light generating system.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1A - 1B schematically depict embodiments of a light generating system according to the invention;
Fig. 2 schematically depicts an embodiment and aspect according to the invention;
Fig. 3 shows some embodiments;
Fig. 4a shows some excitation and emission spectra of suitable phosphors;
Fig. 4b shows some embodiments of spectral power distributions; and
Fig. 5 schematically depicts some applications.

The schematic drawings are not necessarily to scale.

### DETAILED DESCRIPTION OF THE EMBODIMENTS

Traditional chip-on-board devices (COB) are light sources with a dense packaging of (blue) chips, covered with a single phosphor mixture, used in high lumen density applications like spots. However, also two channel COBs (with two individual addressable chip strings) may be desirable, for e.g., tunable white spot applications. In the most cost-effective architecture, on the chips in one of the strings a warm white phosphor mixture is pre-dispensed, after which the complete light emitting area is filled with a second (cool white) phosphor mixture, covering both the warm white and the cool white chip strings. Alternatively, the second phosphor mixture only covers the cool white strings. By tuning the current through the individual strings, different CCTs can be generated. The intermediate CCTs, may be on the line connecting the two primaries (color point of the warm white and cool white string). If the two primaries are located on the BBL, the intermediate color points may be located slightly below BBL.

A third string could be introduced to increase the accessible color gamut, for instance to tune the intermediate color points towards the BBL. This can be done using a green emitter (direct green, or full converted lime) or by using an intermediate 'whitish' color point that is not located on the line connecting the other two primaries (the 3 color points span a triangle in the color space).

Another reason to add a third channel to the COB could be to be able to tune (for example) the α-opic output of the device, independent of CCT. Such a device may be able to generate e.g., 4000K white light with a high melanopic-DER (daytime applications) or low melanopic-DER (evening). Preferably these two settings have the same color point, on or close to the BBL. In order to achieve this, the 3 color points of the individual channels (the primaries) may be located on a single line in the color diagram. Since the tuning towards high MDER may be requested in the cool-white part of the CCT range, 1 of the 3 primaries needed is at low CCT, 2 may be at high CCT. Herein, these primaries may also be indicated as WW (warm white), CW (cool white) (for low MDER) and CP (cyan primary)(cool white) (for high MDER), respectively. The WW and CW primaries may comprise a regular blue chip, the CP primary may comprise a cyan chip to boost the cyan intensity which may be needed to boost MDER.

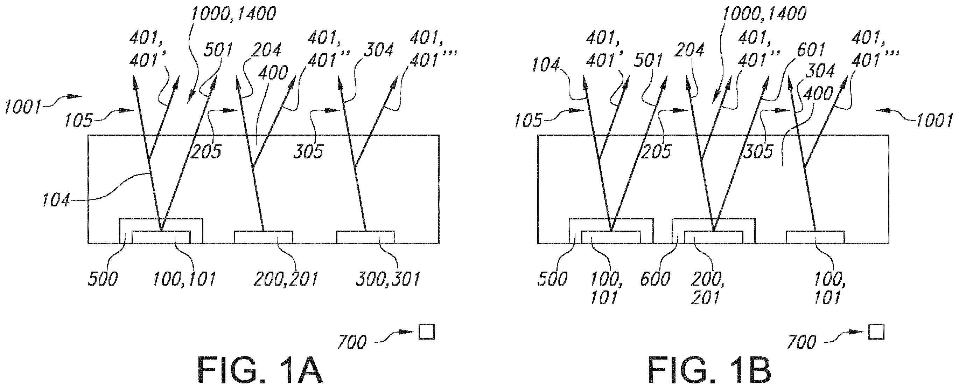

Three color points on a single line in color space can be achieved using different methods. The most straightforward method seems to be tuning of the phosphor for each of the 3 channels. For instance: the warm white channel uses a blue pump and a phosphor pre-dispensing step, similar to the approach nowadays used for a two channel COB, see especially Fig. 1b. In addition, a second pre-dispensing step (using a different phosphor mixture) is applied to the second channel, followed by filling the LES using a third phosphor mixture (see Fig. 1b). In this case three different dispensing steps are needed. This may be an embodiment, but may be less desirable due to process steps with intermediate curing and potential problems with color targeting. Moreover, the chips may need to be spaced further apart as the phosphor deposited on them takes more space than the chip surface area only, leading to larger light-emitting surface (LES), which is undesirable for COBs when used in spots.

Herein, it is especially proposed to cover the two cool white strings with the two different pump wavelengths by a single phosphor mixture. In the approach of Fig. 1a, this is used to fill the LES (light emitting surface) after pre-dispensing on the WW channel, such that the 3-color points align onto a single line in the color space. It was surprisingly found that by proper choice of chip wavelengths and phosphors, it is possible to achieve this, and meet all other desirable spectral requirements (CRI, R9, MDER). The 2nd and 3rd channel have different spectra (different pump) which allows to tune e.g., the melanopic-DER (or other α-opic parameters) independent of CCT (an example is given in Fig. 1).

Hence, in embodiments a tunable melanopic lighting system that allow independent tuning of melanopic-DER and CCT that requires a 3-channel approach is proposed. A 3-channel COB may consists of three individually addressable channels (WW, CW (low MDER), CW (high MDER)). One of the cool white channels uses a blue pump LED, whereas the high MDER channel (CP) uses a cyan pump LED. Both CW channels are comprising the same phosphor. A number of different options were simulated (see further below).

Here below, first Figs. 1-2 are discussed.

Fig. 1A schematically depicts a light generating system 1000 according to the invention. Fig. 1B schematically depicts a light generating system 1000 according to the invention. Referring to Figs. 1A and 1B, the light generating system 1000 may comprise a first SSL light source string 100 comprising one or more first SSL light sources 101 generating blue light, a second SSL light source string 200 comprising one or more second SSL light sources 201 generating blue light, a third SLL light source string 300 comprising one or more third SSL light sources 301 generating cyan light. Referring to Fig. 1A, a first luminescent material 400 may comprise one or more of a yellow, green, yellow/green, orange, red, or orange/red emitting luminescent material. A second luminescent material 400 may comprise one or more of an orange, red, or orange//red emitting luminescent material. Referring to Fig. 1B, a first luminescent material 400 comprises a yellow/green emitting luminescent material, a second luminescent material 400 may comprise an orange, a red or an orange/red emitting luminescent material, and a third luminescent material 400 may comprise a yellow emitting luminescent material. Especially, the luminescent material 400 may comprise a red emitting luminescent material and a green emitting luminescent material. The luminescent material may cover the first SSL light source(s) 101, the second SSL light source(s) 201, and the third SSL light sources 301.

A light generating system, e.g. a chip-on-board (COB) device, with three SSL light source strings allows MDER value tuning substantially independent of the correlated color temperature, and in which the color points of the three SLL light source strings 100, 200, 300 are aligned onto a substantially straight (single) line in color space even though the luminescent material on the second and third SSL light source string may substantially be the same. In other words, there are only two luminescent material application steps. The first luminescent material application can be either on the first SSL light source string 100 or alternatively on the second and third SSL light source strings 200, 300. In case it is on the first SSL light source string 100, the second luminescent material application can be either covering all three SSL light source strings 100, 200, 300 or only the second and third SSL light source strings 200, 300. The color points of the light from the three different SSL light source channels are substantially on a single (straight) line in color space which can be achieved using different methods. The most straightforward method seems to be tuning of the luminescent material for each of the three SLL light source channels. For instance, the first SSL light source string 100 uses one or more blue LEDs and a luminescent material pre-dispensing step. In addition, a second pre-dispensing step, using a different luminescent material, is applied to the second SSL light source string 200, followed by disposing a third luminescent material, see Fig. 1B. In this case three different dispensing steps are needed. Alternatively, the second and third SSL light source strings comprise a substantially identical luminescent material, see Fig. 1A. The first SSL light source string comprises a different luminescent material, see Fig. 1A. The color points of the three SSL light source strings are on a single (straight) line in the color space. By a proper choice of the wavelengths of the SSL light source of the three SSL light source strings and the luminescent material(s), it is possible to achieve this and to obtain desired values for the correlated color temperature and MDER value, amongst others.

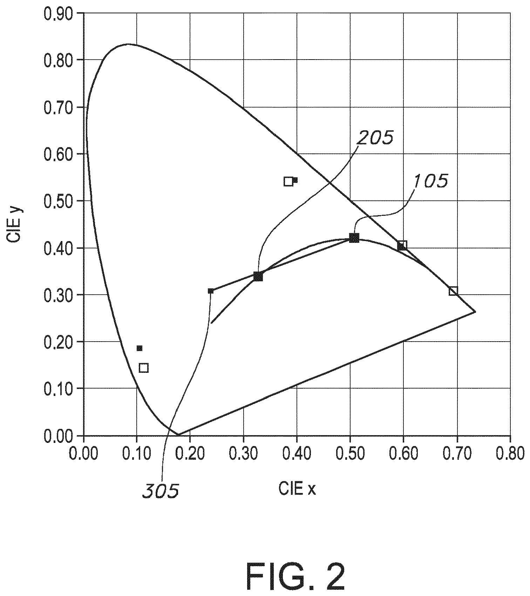

The second and third SSL light source strings 200, 300 may have different spectra which allows to tune the MDER value of the system light substantially independent of the correlated color temperature of the system light, see Fig 2.

The (square) point on the straight line with the lowest x-value, at about 0.23;0.3 may be indicated as CP, wherein "CP" refers to the color point of the third SSL light source string 300; this light is cool white light. The larger (square) point the straight line, at about 0.33;0.33 may be indicated as CW ((also) cool white), wherein "CW" refers to the color point of the second SSL light source string 200. The larger (square) point at the right end of the straight line, at about 0.51;0.41 may be indicated as WW, wherein "WW" refers to the color point of the first SSL light source string 100.

In Fig. 2, the first and second SSL light source strings 100, 200 have one or more blue LEDs with a peak wavelength of 430 nm, the third SSL light source string 300 has one or more cyan LEDs with a dominant wavelength of 477 nm, the light generating system further comprises a BOSE F524/BR2-607a mixture of luminescent materials, such as comprising a (divalent) europium doped silicate and a divalent europium doped nitride.

The tables below show the (maximum) MDER value as a function of correlated color temperature (CCT) and color rendering index (CRI)/R9 combinations, for a light generating system having a first SSL and a second SSL light source string with blue LEDs (peak wavelength 430 nm), and a third SLL light source string with cyan LEDs (dominant wavelength 473 nm), a yellow phosphor (GNYAG3557), such as a cerium doped A₃(Ga,Al)₅O₁₂ garnet (wherein A may especially comprise Y, see also above), and a red phosphor (BR2/607a). In embodiments, the red phosphor may comprise a divalent europium doped nitride.

In embodiments, the first SSL light source string comprises the red phosphor and all SSL light source strings comprise the yellow phosphor. In other embodiments, the first SSL light source string may comprise a (i) yellow and/or green phosphor and (ii) a red phosphor (as fourth luminescent material 500), and all SSL light source strings may comprise a (a) yellow and/or green phosphor and (b) a red phosphor (as first (and/or second and/or third) luminescent material 400). Especially, the first luminescent material 400 may be configured downstream of the fourth luminescent material 500.

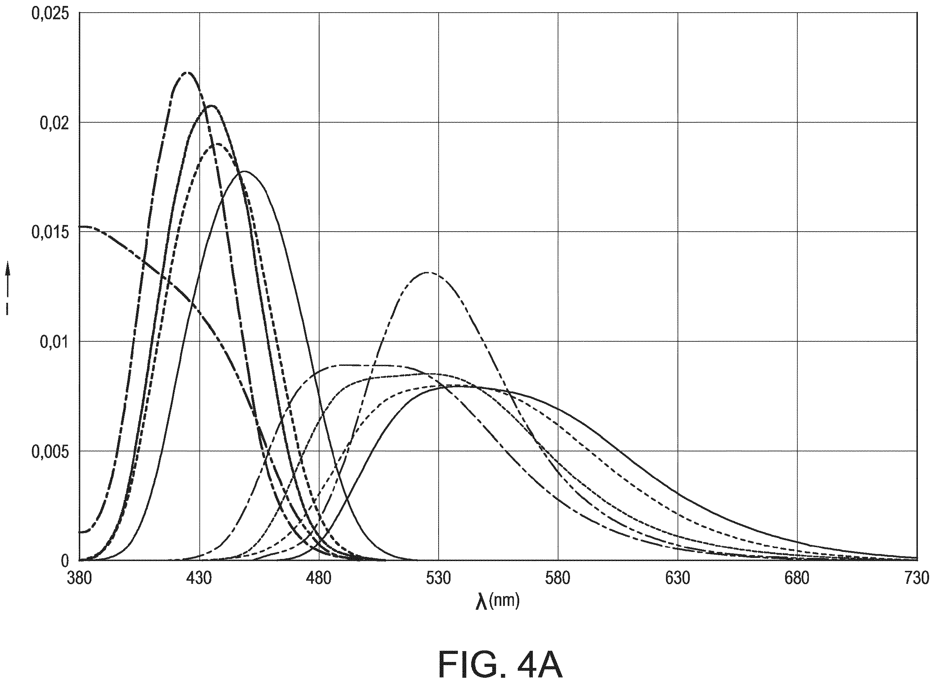

Excitation spectra (thicker lines; shorter wavelength part of the graph) and emission spectra (thinner lines; longer wavelength part of the graph) of suitable luminescent material are depicted in Fig. 4a.

Exemplary simulations started with the selection of the blue pump wavelength (PWL) of the CW channel (low MDER). In the next step the phosphor composition is fixed, in such a way that the desired color point is reached. In the example shown here, the yellow phosphor is a 'normal' garnet phosphor in combination with a short wavelength red emitter (BR2-607a). The target color point is 6500K (above BBL), so that the line connecting the warm white and the cool white primary crosses the BBL at 2200K and 5000K (the requirement). In the last step the cyan wavelength for the third channel is chosen, such that the CP color point is on the same line (line connecting the warm white primary and cool white target point, see Fig. 2). Spectra are generated with different fractions of the three channels (2200K was selected as the warm white channel).

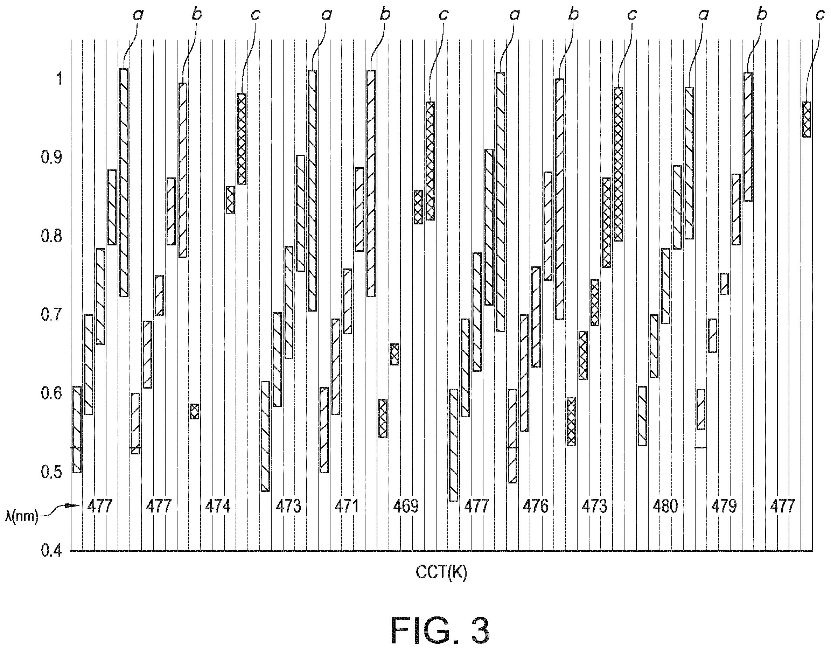

After setting the requirements for CRI and R9 as a function of CCT, the min- and max value of melanopic-DER can be extracted (see below tables). For a certain application the low CCT settings are required to have excellent light quality (CRI>90, R9>50), whereas at higher CCTs the requirements on the light quality are less stringent (5000K, CRI>80). Using these light quality boundary conditions, the results can be summarized in a single graph (Fig. 3).

| **MAX** | **CRI** | **80** | **80** | **85** | | **85** | **90** | **90** | **90** |
|---|---|---|---|---|---|---|---|---|---|
| **MDER** | **R9** | **0** | **50** | **0** | | **50** | **0** | **50** | **80** |
| | **3000** | 0,682 | 0,682 | 0,665 | | 0,665 | 0,614 | 0,614 | 0,614 |
| | **3500** | 0,814 | 0,814 | 0,765 | | 0,765 | 0,702 | 0,702 | 0,702 |
| | **4000** | 0,900 | 0,900 | 0,847 | | 0,847 | 0,787 | 0,787 | 0,787 |
| | **4500** | 0,966 | 0,966 | 0,902 | | 0,902 | 0,856 | 0,856 | 0,856 |
| | **5000** | 1,011 | 1,011 | 0,965 | | 0,965 | 0,889 | 0,889 | |

| **MIN** | **CRI** | **80** | **80** | **85** | **85** | **90** | **90** | **90** |
|---|---|---|---|---|---|---|---|---|
| **MDER** | **R9** | **0** | **50** | **0** | **50** | **0** | **50** | **80** |
| | **3000** | 0,469 | 0,469 | 0,469 | 0,469 | 0,487 | 0,487 | 0,603 |
| | **3500** | 0,529 | 0,555 | 0,529 | 0,555 | 0,579 | 0,579 | 0,684 |
| | **4000** | 0,577 | 0,666 | 0,609 | 0,666 | 0,666 | 0,666 | 0,787 |
| | **4500** | 0,655 | 0,750 | 0,688 | 0,750 | 0,720 | 0,750 | 0,856 |
| | **5000** | 0,695 | 0,832 | 0,768 | 0,832 | 0,801 | 0,832 | |

Fig. 3 summarizes the MDER range that can be reached per green phosphor and blue chip wavelength, and which cyan chip wavelength is needed for that. Four different luminescent materials have been applied, indicated with references P1-P4. The MDER range is given as a function of CCT for the four different phosphors using 435 nm blue LEDs, indicated with bars a, 445 nm, indicated with bars b, and 455 nm indicated with bars c. Light quality requirements may especially be: 2200-4000K: CRI>90, R9>50, 4500K: CRI>85, R9>50, 5000K: CRI>80, R9>0.

Fig. 4a shows excitation and emission spectra of suitable first luminescent materials, second luminescent materials, and third luminescent materials (see also above).

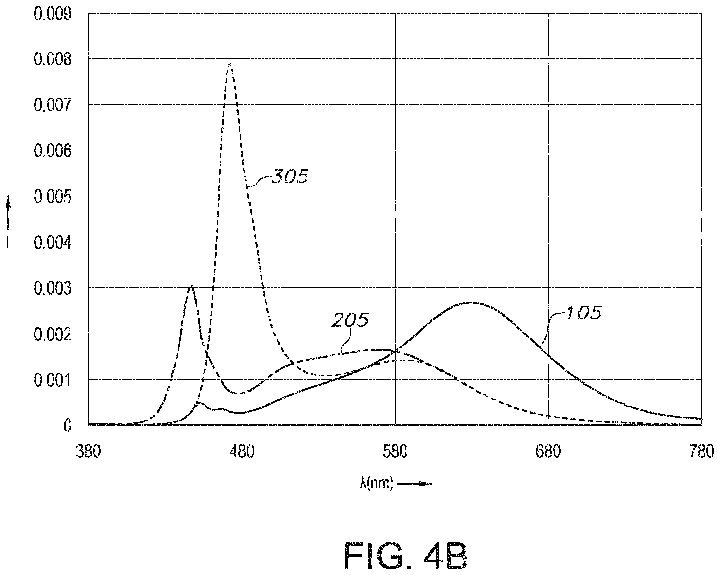

Fig. 4b shows the three light source spectra based on switching at least one first SSL light source, leading to first light 105, at least one second SSL light source, leading to second light 205, at least one third SSL light source, leading to third light 305.

The color point of the first light 105 is (2° CMF): CIE u' 0.2890, CIE v' 0.5365; the color point of the second light 205 is (2° CMF): CIE u' 0.1942, CIE v' 0.4787; the color point of the third light 305 is (2° CMF): CIE u' 0.1746, CIE v' 0.4359; see also the table below:

| | 105 | 205 | 305 |
|---|---|---|---|
| u'(2) | 0.289 | 0.194 | 0.175 |
| v'(2) | 0.537 | 0.470 | 0.436 |
| u'(10) | 0.294 | 0.200 | 0.164 |
| v'(10) | 0.536 | 0.478 | 0.455 |
| x10 | 0.510 | 0.324 | 0.259 |
| y10 | 0.414 | 0.345 | 0.318 |

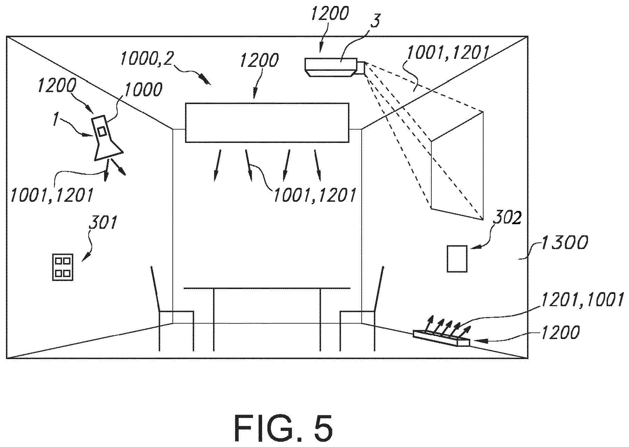

Fig. 5 schematically depicts an embodiment of a luminaire 2 comprising the light generating system 1000 as described above. Reference 301 indicates a user interface which may be functionally coupled with the control system 302 comprised by or functionally coupled to the light generating system 1000. Fig. 5 also schematically depicts an embodiment of lamp 1 comprising the light generating system 1000. Reference 3 indicates a projector device or projector system, which may be used to project images, such as at a wall, which may also comprise the light generating system 1000. Hence, Fig. 5 schematically depicts embodiments of a lighting device 1200 selected from the group of a lamp 1, a luminaire 2, a projector device 3, a disinfection device, a photochemical reactor, and an optical wireless communication device, comprising the light generating system 1000 as described herein. In embodiments, such lighting device may be a lamp 1, a luminaire 2, a projector device 3, a disinfection device, or an optical wireless communication device. Lighting device light escaping from the lighting device 1200 is indicated with reference 1201. Lighting device light 1201 may essentially consist of system light 1001, and may in specific embodiments thus be system light 1001.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

**In** the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. **In** a device claim, or an apparatus claim, or a system claim, enumerating several means, several of these means may be embodied by one and the same item of hardware.

## Claims

1. A light generating system (1000), configured to generate system light (1001), wherein the light generating system (1000) comprises a first SSL light source string (100), a second SSL light source string (200) and a third SSL light source string (300), wherein:
- the first SSL light source string (100) is configured to generate first light (105) having a first color point and a first correlated color temperature CCT1, and which comprises at least one first SSL light source (101) for generating violet and/or blue light and a first luminescent material (400),
- the second SSL light source string (200) is configured to generate second light (205) having a second color point and a second correlated color temperature CCT2, and which comprises at least one second SSL light source (201) for generating violet and/or blue light and a second luminescent material (400),
- the third SSL light source string (300) is configured to generate third light (305) having a third color point and a third correlated color temperature CCT3, and which comprises at least one third SSL light source (301) for generating cyan light and a third luminescent material (400),
- the first correlated color temperature is lower than the second correlated color temperature,
- the first correlated color temperature is lower than the third correlated color temperature;
- the first SSL light source string (100) is configured to generate warm-white light, and wherein the second SSL light source string (200) and the third SSL light source string (300) are configured to generate cold-white light;
- the first color point, the second color point, and the third color point are on a straight line in a color diagram, and wherein the first color point, the second color point and the third color point do not overlap within 10 standard deviation of color matching, SDCM;
- wherein the first SSL light source string (100), the second SSL light source string (200) and the third SSL light source string (300) are individually controllable; and
- wherein the system light (1001) is substantially white light with a correlated color temperature in the range between 2200 - 6500 K.

2. The light generating system (1000) according to claim 1, wherein the first SSL light source (101) is configured to generate first SSL light (104) having a first dominant wavelength λ1d selected from the range of 440-470 nm, wherein the second SSL light source (201) is configured to generate second SSL light (204) having a second dominant wavelength λ2d selected from the range of 425-470 nm, and wherein the third SSL light source (301) is configured to generate third SSL light (304) having a third dominant wavelength λ3d selected from the range of 470-520 ; wherein (i) λ3d>λ2d and λ3d>λ1d, and/or (ii) λ3c>λ2c and λ3c>λ1c.

3. The light generating system (1000) according to any of the preceding claims, wherein the first light (105) has a first Melanopic Daylight Efficacy Ratio, MDER, wherein the second light (205) has a second Melanopic Daylight Efficacy Ratio, MDER, and wherein the third light has a third Melanopic Daylight Efficacy Ratio, MDER, wherein the second MDER value is lower than the third MDER value.

4. The light generating system (1000) according to claim 3, wherein the third MDER value is larger than both the first MDER value and the second MDER value; wherein at least two of the first MDER value, the second MDER value, and the third MDER value mutually differ at least 0.1.

5. The light generating system (1000) according to any one of the preceding claims, further comprising a control system (302) configured to individually control the first SSL light source string (100), the second SSL light source string (200), and the third SSL light source string (300).

6. The light generating system (1000) according to any one of the preceding claims, wherein the first luminescent material and/or the second luminescent material and/or the third luminescent material comprises a luminescent material of the type A₃B₅O₁₂:Ce, wherein A comprises one or more of Y, La, Gd, Tb and Lu, and wherein B comprises one or more of Al, Ga, In and Sc.

7. **The light** generating system (1000) according to any one of the preceding claims, wherein the first luminescent material, second luminescent material, and third luminescent material are substantially identical.

8. The light generating system (1000) according to any one of the preceding claims, further comprising a fourth luminescent material (500) different from the first, second and third luminescent material, wherein the fourth luminescent material (500) is configured downstream of the at least one first SSL light source (101) and is not configured downstream of the at least one second SSL light source (201) and the at least one third SSL light source (301).

9. The light generating system (1000) according to any one of the preceding claims 7-8, wherein the first luminescent material, the second luminescent material, and third luminescent material are configured downstream of all of the at least one first SSL light source (101), the at least one second SSL light source (201), and the at least one third SSL light source (301), and wherein there is no other luminescent material configured downstream of the at least one second SSL source (201) and the at least one third SSL light source (301).

10. The light generating system (1000) according to any one of the preceding claims 7-9, wherein the second SSL light source string further comprises a fifth luminescent material (600).

11. The light generating system (1000) according to any one of the preceding claims 7-10, wherein the fourth luminescent material is an orange and/or red light emitting luminescent material.

12. The light generating system (1000) according to any one of the preceding claims, wherein the third correlated color temperature CCT3 > 3000 K, the second correlated color temperature CCT2 > 3000 K, and the first correlated color temperature CCT1 ≤ 2700 K.

13. The light generating system (1000) according to any one of the preceding claims, wherein the first correlated color temperature differs from the second correlated color temperature and the third correlated color temperature with at least 1000 K, wherein all three color points, determined on the basis of 10° color matching functions are positioned within 10 standard deviation of color matching, SDCM of the substantially straight line in the color diagram.

14. The light generating system (1000) according to any one of the preceding claims, comprising a chip-on-board device (1400), wherein the chip-on-board-device (1400) comprises the first SSL light source string (100), the second SSL light source string (200), the third SSL light source string (300), the first luminescent material (400), the second luminescent material (400), and the third luminescent material (400).

15. A lighting device (1200) selected from the group of a lamp, a luminaire, and a projector device, comprising the light generating system (1000) according to any one of the preceding claims.

## Patentansprüche

1. Lichterzeugungssystem (1000), das konfiguriert ist, um Systemlicht (1001) zu erzeugen, wobei das Lichterzeugungssystem (1000) einen ersten SSL-Lichtquellenstrang (100), einen zweiten SSL-Lichtquellenstrang (200) und einen dritten SSL-Lichtquellenstrang (300) umfasst, wobei:
- der erste SSL-Lichtquellenstrang (100) konfiguriert ist, um erstes Licht (105), das einen ersten Farbpunkt und eine erste korrelierte Farbtemperatur CCT1 aufweist, zu erzeugen, und wobei er mindestens eine erste SSL-Lichtquelle (101) zum Erzeugen violetten und/oder blauen Lichts und einen ersten Leuchtstoff (400) umfasst,
- der zweite SSL-Lichtquellenstrang (200) konfiguriert ist, um zweites Licht (205), das einen zweiten Farbpunkt und eine zweite korrelierte Farbtemperatur CCT2 aufweist, zu erzeugen, und wobei er mindestens eine zweite SSL-Lichtquelle (201) zum Erzeugen violetten und/oder blauen Lichts und einen zweiten Leuchtstoff (400) umfasst,
- der dritte SSL-Lichtquellenstrang (300) konfiguriert ist, um drittes Licht (305), das einen dritten Farbpunkt und eine dritte korrelierte Farbtemperatur CCT3 aufweist, zu erzeugen, und wobei er mindestens eine dritte SSL-Lichtquelle (301) zum Erzeugen von Cyanlicht und einen dritten Leuchtstoff (400) umfasst,
- die erste korrelierte Farbtemperatur niedriger als die zweite korrelierte Farbtemperatur ist,
- die erste korrelierte Farbtemperatur niedriger als die dritte korrelierte Farbtemperatur ist;
- der erste SSL-Lichtquellenstrang (100) konfiguriert ist, um warmweißes Licht zu erzeugen, und wobei der zweite SSL-Lichtquellenstrang (200) und der dritte SSL-Lichtquellenstrang (300) konfiguriert sind, um kaltweißes Licht zu erzeugen;
- der erste Farbpunkt, der zweite Farbpunkt und der dritte Farbpunkt auf einer geraden Linie in einem Farbdiagramm liegen, und wobei der erste Farbpunkt, der zweite Farbpunkt und der dritte Farbpunkt sich innerhalb von 10 Standard Deviation of Color Matching, SDCM, nicht überlappen;
- wobei der erste SSL-Lichtquellenstrang (100), der zweite SSL-Lichtquellenstrang (200) und der dritte SSL-Lichtquellenstrang (300) einzeln steuerbar sind; und
- wobei das Systemlicht (1001) im Wesentlichen weißes Licht mit einer korrelierten Farbtemperatur in dem Bereich zwischen 2200-6500 K ist.

2. Lichterzeugungssystem (1000) nach Anspruch 1, wobei die erste SSL-Lichtquelle (101) konfiguriert ist, um erstes SSL-Licht (104) zu erzeugen, das eine erste farbtongleiche Wellenlänge λ1d, die aus dem Bereich von 440-470 nm ausgewählt ist, aufweist, wobei die zweite SSL-Lichtquelle (201) konfiguriert ist, um zweites SSL-Licht (204) zu erzeugen, das eine zweite farbtongleiche Wellenlänge λ2d, die aus dem Bereich von 425-470 nm ausgewählt ist, aufweist, und wobei die dritte SSL-Lichtquelle (301) konfiguriert ist, um drittes SSL-Licht (304) zu erzeugen, das eine dritte farbtongleiche Wellenlänge λ3d, die aus dem Bereich von 470-520 ausgewählt ist, aufweist; wobei (i) λ3d>λ2d und λ3d>λ1d, und/oder (ii) λ3c>λ2c und λ3c>λ1c.

3. Lichterzeugungssystem (1000) nach einem der vorstehenden Ansprüche, wobei das erste Licht (105) einen ersten melanopischen Wirkfaktor, MDER, aufweist, wobei
das zweite Licht (205) einen zweiten melanopischen Wirkfaktor, MDER, aufweist, und
wobei das dritte Licht einen dritten melanopischen Wirkfaktor, MDER, aufweist, wobei der zweite MDER-Wert niedriger ist als der dritte MDER-Wert ist.

4. Lichterzeugungssystem (1000) nach Anspruch 3, wobei der dritte MDER-Wert größer als sowohl der erste MDER-Wert als auch der zweite MDER-Wert ist; wobei sich mindestens zwei von dem ersten MDER-Wert, dem zweiten MDER-Wert und dem dritten MDER-Wert um mindestens 0,1 voneinander unterscheiden.

5. Lichterzeugungssystem (1000) nach einem der vorstehenden Ansprüche, ferner umfassend ein Steuersystem (302), das konfiguriert ist, um den ersten SSL-Lichtquellenstrang (100), den zweiten SSL-Lichtquellenstrang (200) und den dritten SSL-Lichtquellenstrang (300) einzeln zu steuern.

6. Lichterzeugungssystem (1000) nach einem der vorstehenden Ansprüche, wobei der erste Leuchtstoff und/oder der zweite Leuchtstoff und/oder der dritte Leuchtstoff einen Leuchtstoff von der Art A₃B₅O₁₂:Ce umfasst, wobei A eines oder mehrere von Y, La, Gd, Tb und Lu umfasst, und wobei B eines oder mehrere von Al, Ga, In und Sc umfasst.

7. Lichterzeugungssystem (1000) nach einem der vorstehenden Ansprüche, wobei der erste Leuchtstoff, der zweite Leuchtstoff und der dritte Leuchtstoff im Wesentlichen identisch sind.

8. Lichterzeugungssystem (1000) nach einem der vorstehenden Ansprüche, ferner umfassend einen vierten Leuchtstoff (500), der sich von dem ersten, zweiten und dritten Leuchtstoff unterscheidet, wobei der vierte Leuchtstoff (500) stromabwärts der mindestens einen ersten SSL-Lichtquelle (101) konfiguriert ist und nicht stromabwärts der mindestens einen zweiten SSL-Lichtquelle (201) und der mindestens einen dritten SSL-Lichtquelle (301) konfiguriert ist.

9. Lichterzeugungssystem (1000) nach einem der vorstehenden Ansprüche 7 bis 8, wobei der erste Leuchtstoff, der zweite Leuchtstoff und der dritte Leuchtstoff stromabwärts von allen der mindestens einen ersten SSL-Lichtquelle (101), der mindestens einen zweiten SSL-Lichtquelle (201) und der mindestens einen dritten SSL-Lichtquelle (301) konfiguriert sind, und wobei kein anderer Leuchtstoff stromabwärts der mindestens einen zweiten SSL-Quelle (201) und der mindestens einen dritten SSL-Lichtquelle (301) konfiguriert ist.

10. Lichterzeugungssystem (1000) nach einem der vorstehenden Ansprüche 7 bis 9, wobei der zweite SSL-Lichtquellenstrang ferner einen fünften Leuchtstoff (600) umfasst.

11. Lichterzeugungssystem (1000) nach einem der vorstehenden Ansprüche 7 bis 10, wobei der vierte Leuchtstoff ein Leuchtstoff ist, der oranges und/oder rotes Licht emittiert.

12. Lichterzeugungssystem (1000) nach einem der vorstehenden Ansprüche, wobei die dritte korrelierte Farbtemperatur CCT3 > 3000 K, die zweite korrelierte Farbtemperatur CCT2 > 3 000 K und die erste korrelierte Farbtemperatur CCT1 ≤ 2700 K ist.

13. Lichterzeugungssystem (1000) nach einem der vorstehenden Ansprüche, wobei sich die erste korrelierte Farbtemperatur von der zweiten korrelierten Farbtemperatur und der dritten korrelierten Farbtemperatur um mindestens 1000 K unterscheidet, wobei alle drei Farbpunkte, bestimmt auf der Basis von 10°-Spektralwertfunktionen, innerhalb von 10 Standard Deviation of Color Matching, SDCM, der im Wesentlichen geraden Linie in dem Farbdiagramm positioniert sind.

14. Lichterzeugungssystem (1000) nach einem der vorstehenden Ansprüche, umfassend ein Chip-on-Board-Bauelement (1400), wobei das Chip-on-Board-Bauelement (1400) den ersten SSL-Lichtquellenstrang (100), den zweiten SSL-Lichtquellenstrang (200), den dritten SSL-Lichtquellenstrang (300), den ersten Leuchtstoff (400), den zweiten Leuchtstoff (400) und den dritten Leuchtstoff (400) umfasst.

15. Beleuchtungsvorrichtung (1200), die aus der Gruppe einer Lampe, einer Leuchte und einer Projektorvorrichtung, umfassend das Lichterzeugungssystem (1000) nach einem der vorstehenden Ansprüche, ausgewählt ist.

## Revendications

1. Système générateur de lumière (1000), configuré pour générer de la lumière de système (1001), dans lequel le système générateur de lumière (1000) comprend un premier chapelet de sources de lumière SSL (100), un deuxième chapelet de sources de lumière SSL (200) et un troisième chapelet de sources de lumière SSL (300), dans lequel :
- le premier chapelet de sources de lumière SSL (100) est configuré pour générer de la première lumière (105) ayant un premier point de couleur et une première température de couleur corrélée CCT1, et qui comprend au moins une première source de lumière SSL (101) permettant de générer de la lumière violette et/ou bleue et un premier matériau luminescent (400),
- le deuxième chapelet de sources de lumière SSL (200) est configuré pour générer de la deuxième lumière (205) ayant un deuxième point de couleur et une deuxième température de couleur corrélée CCT2, et qui comprend au moins une deuxième source de lumière SSL (201) permettant de générer de la lumière violette et/ou bleue et un deuxième matériau luminescent (400),
- le troisième chapelet de sources de lumière SSL (300) est configuré pour générer de la troisième lumière (305) ayant un troisième point de couleur et une troisième température de couleur corrélée CCT3, et qui comprend au moins une troisième source de lumière SSL (301) permettant de générer de la lumière cyan et un troisième matériau luminescent (400),
- la première température de couleur corrélée est inférieure à la deuxième température de couleur corrélée,
- la première température de couleur corrélée est inférieure à la troisième température de couleur corrélée ;
- le premier chapelet de sources de lumière SSL (100) est configuré pour générer de la lumière blanche chaude, et dans lequel le deuxième chapelet de sources de lumière SSL (200) et le troisième chapelet de sources de lumière SSL (300) sont configurés pour générer de la lumière blanche froide ;
- le premier point de couleur, le deuxième point de couleur et le troisième point de couleur sont sur une ligne droite dans un diagramme de couleur, et dans lequel le premier point de couleur, le deuxième point de couleur et le troisième point de couleur ne se chevauchent pas à plus ou moins 10 écarts-types de correspondance de couleur, SDCM ;
- dans lequel le premier chapelet de sources de lumière SSL (100), le deuxième chapelet de sources de lumière SSL (200) et le troisième chapelet de sources de lumière SSL (300) peuvent être commandés individuellement ; et
- dans lequel la lumière de système (1001) est de la lumière sensiblement blanche avec une température de couleur corrélée dans la plage entre 2200 et 6500 K.

2. Système générateur de lumière (1000) selon la revendication 1, dans lequel la première source de lumière SSL (101) est configurée pour générer de la première lumière SSL (104) ayant une première longueur d'onde dominante λ1d sélectionnée dans la plage de 440 à 470 nm, dans lequel la deuxième source de lumière SSL (201) est configurée pour générer de la deuxième lumière SSL (204) ayant une deuxième longueur d'onde dominante λ2d sélectionnée dans la plage de 425 à 470 nm, et dans lequel la troisième source de lumière SSL (301) est configurée pour générer de la troisième lumière SSL (304) ayant une troisième longueur d'onde dominante λ3d sélectionnée dans la plage de 470 à 520 ; dans lequel (i) λ3d>λ2d et λ3d>λ1d, et/ou (ii) λ3c>λ2c et λ3c>λ1c.

3. Système générateur de lumière (1000) selon l'une quelconque des revendications précédentes, dans lequel la première lumière (105) a un premier rapport d'efficacité de lumière du jour mélanopique, MDER, dans lequel
la deuxième lumière (205) a un deuxième rapport d'efficacité de lumière du jour mélanopique, MDER, et
dans lequel la troisième lumière a un troisième rapport d'efficacité de lumière du jour mélanopique, MDER, dans lequel la deuxième valeur MDER est inférieure à la troisième valeur MDER.

4. Système générateur de lumière (1000) selon la revendication 3, dans lequel la troisième valeur MDER est plus grande à la fois que la première valeur MDER et que la deuxième valeur MDER ; dans lequel au moins deux parmi la première valeur MDER, la deuxième valeur MDER et la troisième valeur MDER diffèrent mutuellement d'au moins 0,1.

5. Système générateur de lumière (1000) selon l'une quelconque des revendications précédentes, comprenant en outre un système de commande (302) configuré pour commander individuellement le premier chapelet de sources de lumière SSL (100), le deuxième chapelet de sources de lumière SSL (200) et le troisième chapelet de sources de lumière SSL (300).

6. Système générateur de lumière (1000) selon l'une quelconque des revendications précédentes, dans lequel le premier matériau luminescent et/ou le deuxième matériau luminescent et/ou le troisième matériau luminescent comprennent un matériau luminescent du type A₃B₅O₁₂:Ce, dans lequel A comprend un ou plusieurs parmi Y, La, Gd, Tb et Lu, et dans lequel B comprend un ou plusieurs parmi AI, Ga, In et Sc.

7. Système générateur de lumière (1000) selon l'une quelconque des revendications précédentes, dans lequel le premier matériau luminescent, le deuxième matériau luminescent et le troisième matériau luminescent sont sensiblement identiques.

8. Système générateur de lumière (1000) selon l'une quelconque des revendications précédentes, comprenant en outre un quatrième matériau luminescent (500) différent des premier, deuxième et troisième matériaux luminescents, dans lequel le quatrième matériau luminescent (500) est configuré en aval de l'au moins une première source de lumière SSL (101) et n'est pas configuré en aval de l'au moins une deuxième source de lumière SSL (201) et de l'au moins une troisième source de lumière SSL (301).

9. Système générateur de lumière (1000) selon l'une quelconque des revendications 7 à 8 précédentes, dans lequel le premier matériau luminescent, le deuxième matériau luminescent et le troisième matériau luminescent sont configurés en aval de toutes parmi l'au moins une première source de lumière SSL (101), l'au moins une deuxième source de lumière SSL (201) et l'au moins une troisième source de lumière SSL (301), et dans lequel il n'y a aucun autre matériau luminescent configuré en aval de l'au moins une deuxième source SSL (201) et de l'au moins une troisième source de lumière SSL (301).

10. Système générateur de lumière (1000) selon l'une quelconque des revendications 7 à 9 précédentes, dans lequel le deuxième chapelet de sources de lumière SSL comprend en outre un cinquième matériau luminescent (600).

11. Système générateur de lumière (1000) selon l'une quelconque des revendications 7 à 10 précédentes, dans lequel le quatrième matériau luminescent est un matériau luminescent émettant de la lumière orange et/ou rouge.

12. Système générateur de lumière (1000) selon l'une quelconque des revendications précédentes, dans lequel la troisième température de couleur corrélée CCT3 > 3000 K, la deuxième température de couleur corrélée CCT2 > 3000 K, et la première température de couleur corrélée CCT1 ≤ 2700 K.

13. Système générateur de lumière (1000) selon l'une quelconque des revendications précédentes, dans lequel la première température de couleur corrélée diffère de la deuxième température de couleur corrélée et de la troisième température de couleur corrélée d'au moins 1000 K, dans lequel tous les trois points de couleur, déterminés sur la base de fonctions de correspondance de couleurs à 10° sont positionnés à plus ou moins 10 écarts-types de correspondance de couleur, SDCM de la ligne sensiblement droite dans le diagramme de couleur.

14. Système générateur de lumière (1000) selon l'une quelconque des revendications précédentes, comprenant un dispositif de puce sur carte (1400), dans lequel le dispositif de puce sur carte (1400) comprend le premier chapelet de sources de lumière SSL (100), le deuxième chapelet de sources de lumière SSL (200), le troisième chapelet de sources de lumière SSL (300), le premier matériau luminescent (400), le deuxième matériau luminescent (400) et le troisième matériau luminescent (400).

15. Dispositif d'éclairage (1200) choisi dans le groupe d'une lampe, d'un luminaire, d'un dispositif projecteur, comprenant le système générateur de lumière (1000) selon l'une quelconque des revendications précédentes.
